# NEW EUROPEAN PATENT SPECIFICATION

(11) **EP 2 846 758 B2**
(45) Date of publication and mention of the opposition decision: **21.05.2025**
(45) Mention of the grant of the patent: 22.11.2017
(21) Application number: 13722366.5
(22) Date of filing: 06.05.2013
(51) Int. Cl.: A61K 8/04, A61K 8/19, A61K 8/81, A61Q 5/06, A61K 8/02

(54) **AEROSOL DEVICE BASED ON A CALCIUM SALT AND A FIXING POLYMER**
AEROSOLVORRICHTUNG AUF DER BASIS EINES CALCIUMSALZES UND EIN FIXIERENDES POLYMER
DISPOSITIF D'AÉROSOL À BASE DE SEL DE CALCIUM ET POLYMÈRE FIXANT

(30) Priority: 07.05.2012 FR 1254168; 29.06.2012 US 201261666783 P
(43) Date of publication of application: 18.03.2015
(73) Proprietor: L'OREAL, 75008 Paris (FR)
(72) Inventor: SMAIL, Nadia, F-78540 Vernouillet (FR); GAWTREY, Jonathan, F-92100 Boulogne (FR)
(74) Representative: Casalonga
(86) International application number: PCT/EP2013/059382
(87) International publication number: WO 2013/167530

(56) References cited:
- EP-A1- 1 026 220
- WO-A1-02/096379
- DE-A1- 102006 045 964
- DE-A1- 102009 054 978
- FR-A1- 2 715 841
- FR-A1- 2 924 341
- US-A- 4 983 377
- US-A1- 2004 170 575
- "Styling Mousse", GNPD; MINTEL, 1 November 2008 (2008-11-01), XP002736036
- DATABASE WPI Week 201172, Derwent World Patents Index; AN 2011-N36295, XP002690571

## Description

The present invention relates to an aerosol device comprising a cosmetic composition based on at least one water-insoluble calcium salt and at least one fixing polymer, and to the use thereof for the cosmetic treatment of the hair, especially for shaping the hair and/or holding the hairstyle.

The hair products for shaping and/or holding the hairstyle that are the most widespread on the cosmetics market are spray compositions, such as lacquers and sprays. They are essentially formed from an alcoholic or aqueous solution and from one or more materials, generally polymer resins, also known as fixing components, whose function is to form welds between the hairs, as a mixture with various cosmetic adjuvants. An aerosol device for hair styling comprising a cosmetic composition is described for example in WO 02/096379. It comprises solid particles such as silicate particles, a fixing polymer, ethanol and a propellant. These products provide fixing and hold of the hairstyle over time, but they have a tendency to set the head of hair, giving the impression of having a helmet, known as the helmet effect. This criterion is often negatively perceived by consumers.

In the course of the day, if the user passes his hands through his hair, the fixing provided by the lacquer is reduced. At the present time, the polymers conventionally used do not make it possible to re-establish the shape of the hairstyle if it has been disrupted.

The volume of the hairstyle also has a tendency to decrease greatly in the course of the day, particularly in the case of fine hair.

There is thus a need to propose fixing products that do not set the head of hair and that can re-establish the shape of the hairstyle.

The Applicant has found, surprisingly and advantageously, that the combination of a particular amount of a water-insoluble calcium salt, whose mean particle size preferably ranges from 2 µm to 50 µm, the water-insoluble calcium salts including calcium carbonate and/or calcium stearate, with a fixing polymer in an aerosol medium makes it possible to obtain a hairstyle with volume and texturizing, while at the same time having the possibility of reworking the hairstyle.

One subject of the invention is thus an aerosol device containing a cosmetic composition comprising:
(i) from 0.1% to 15% by weight relative to the total weight of the composition, of one or more water-insoluble calcium salts, the one or more water-insoluble calcium salts including calcium carbonate and/or calcium stearate,
(ii) one or more fixing polymers,
(iii) one or more C₂₋₄ monoalcohols, and
(iv) one or more propellants.

This particular combination allows shaping and/or hold of the hairstyle by texturizing without setting it.

It also allows re-establishment of the shape of the hairstyle even after it has been intentionally or unintentionally modified. The hairstyle can also be remodelled in the course of the day, without additional application of product.

This combination also has a particularly noteworthy effect on the volume of the hairstyle.

A significant decrease in the white effect perceived on the hair with products based on solid particles is also obtained.

The application of the cosmetic composition according to the invention may equally be performed on wet or dry hair.

The present invention also relates to a cosmetic treatment process, especially a process for shaping the hair and/or for holding the hairstyle, comprising the spraying onto the hair of the composition according to the invention.

A subject of the invention is also the use of the cosmetic composition sprayed from this aerosol device, for shaping the hair and/or holding the hairstyle, and especially for giving the hairstyle volume.

Other subjects, characteristics, aspects and advantages of the invention will become even more clearly apparent on reading the description and the example that follows.

In that which follows and unless otherwise indicated, the limits of a range of values are included within this range, in particular in the expressions "between" and "ranging from ... to ...".

Moreover, the expression "at least one" used in the present description is equivalent to the expression "one or more".

According to the invention, the aerosol device contains a cosmetic composition comprising:
(i) from 0.1% to 15% by weight relative to the total weight of the composition, of one or more water-insoluble calcium salts, the one or more water-insoluble calcium salts including calcium carbonate and/or calcium stearate,
(ii) one or more fixing polymers,
(iii) one or more C₂₋₄ monoalcohols, and
(iv) one or more propellants.

For the purposes of the present invention, the term "water-insoluble" refers to a compound whose solubility at spontaneous pH in water at 25°C and at atmospheric pressure is less than 0.1%.

The calcium salt may be mineral or organic. It is preferably mineral.

Water-insoluble calcium salts include calcium carbonate and calcium stearate. The calcium salt is preferably calcium carbonate.

The water-insoluble calcium salt is in particular in the form of a powder comprising particles preferably with a mean diameter from 2 to 50 µm, preferably from 5 to 40 µm, and better still of about 30 µm.

The calcium salt(s) are present in an amount ranging from 0.1% to 15% by weight, preferably from 0.5% to 10% by weight and even more preferentially from 1% to 5% by weight relative to the total weight of the composition.

As indicated previously, the cosmetic composition according to the invention comprises one or more fixing polymers.

The term "fixing polymer" means any polymer capable of conferring a shape on a head of hair or of maintaining a head of hair in a given shape.

The fixing polymer(s) used in the aerosol device according to the invention are selected from anionic, cationic, amphoteric and nonionic fixing polymers, and mixtures thereof.

Anionic polymers that may be mentioned include polymers comprising groups derived from carboxylic acids, sulfonic acids or phosphoric acids, and having a number-average molecular mass of between 500 and 5 000 000.

The carboxylic groups are provided by unsaturated monocarboxylic or dicarboxylic acid monomers such as those corresponding to the formula: 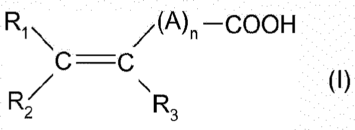 in which n is an integer from 0 to 10, A denotes a methylene group, optionally linked to the carbon atom of the unsaturated group or to the neighbouring methylene group when n is greater than 1 via a heteroatom such as oxygen or sulfur, R₁ denotes a hydrogen atom or a phenyl or benzyl group, R₂ denotes a hydrogen atom, an alkyl group comprising from 1 to 4 carbon atoms or a carboxyl group, and R₃ denotes a hydrogen atom, an alkyl group comprising from 1 to 4 carbon atoms or a -CH₂-COOH, phenyl or benzyl group.

In formula (I) above, the alkyl group comprising from 1 to 4 carbon atoms may in particular denote methyl and ethyl groups.

The anionic fixing polymers containing carboxylic or sulfonic groups that are preferred are:
A) copolymers of acrylic or methacrylic acid or salts thereof, including copolymers of acrylic acid and of acrylamide and methacrylic acid/acrylic acid/ethyl acrylate/methyl methacrylate copolymers, in particular Amerhold DR 25 sold by the company Amerchol, and the sodium salts of polyhydroxycarboxylic acids. Mention may also be made of methacrylic acid/ethyl acrylate copolymers, especially in aqueous dispersion, such as Luviflex Soft and Luvimer MAE sold by the company BASF;
B) copolymers of acrylic or methacrylic acids with a monoethylenic monomer such as ethylene, styrene, vinyl esters and acrylic or methacrylic acid esters, optionally grafted onto a polyalkylene glycol such as polyethylene glycol and optionally crosslinked. Such polymers are described in particular in French patent 1 222 944 and German patent application 2 330 956, the copolymers of this type comprising an optionally N-alkylated and/or hydroxyalkylated acrylamide unit in their chain as described especially in Luxembourg patent applications 75370 and 75371. Mention may also be made of copolymers of acrylic acid and of a C₁-C₄ alkyl methacrylate.
   As another anionic fixing polymer of this family, mention may also be made of the butyl acrylate/acrylic acid/methacrylic acid branched block anionic polymer sold under the name Fixate G-100 L by the company Lubrizol (INCI name: AMP-acylates/allyl methacrylate copolymer).
C) crotonic acid-based copolymers, such as those comprising in their chain vinyl acetate or propionate units and optionally other monomers such as allylic or methallylic esters, vinyl ether or vinyl ester of a saturated, linear or branched carboxylic acid containing a long hydrocarbon-based chain such as those comprising at least 5 carbon atoms, these polymers possibly being grafted and crosslinked, or alternatively a vinyl, allylic or methallylic ester of an α- or β-cyclic carboxylic acid. Such polymers are described, *inter alia,* in French patents 1 222 944,1 580 545, 2 265 782, 2 265 781, 1 564 110 and 2 439 798. Commercial products that fall within this category are the resins 28-29-30, 26-13-14 and 28-13-10 sold by the company National Starch.
   Crotonic acid-based copolymers that may also be mentioned include crotonic acid/vinyl acetate/vinyl tert-butylbenzoate terpolymers and in particular Mexomer PW supplied by the company Chimex.
D) polymers derived from maleic, fumaric or itaconic acids or anhydrides with vinyl esters, vinyl ethers, vinyl halides, phenylvinyl derivatives or acrylic acid and esters thereof; these polymers may be esterified. Such polymers are described in particular in US patents 2 047 398, 2 723 248 and 2 102 113 and GB patent 839 805, and especially those sold under the names Gantrez^{®} AN or ES by the company ISP.
   Polymers also falling within this category are the copolymers of maleic, citraconic or itaconic anhydrides and of an allylic or methallylic ester optionally comprising an acrylamide or methacrylamide group, an α-olefin, acrylic or methacrylic esters, acrylic or methacrylic acids or vinylpyrrolidone in their chain, the anhydride functions being monoesterified or monoamidated. These polymers are described, for example, in French patents 2 350 384 and 2 357 241 by the Applicant.
E) polyacrylamides comprising carboxylate groups;
F) polymers comprising sulfonic groups. These polymers may be polymers comprising vinylsulfonic, styrenesulfonic, naphthalenesulfonic, acrylamidoalkylsulfonic or sulfoisophthalate units.
   These polymers can be chosen in particular from:
   - polyvinylsulfonic acid salts with a molecular mass of between about 1000 and 100 000, and also copolymers with an unsaturated comonomer such as acrylic or methacrylic acids and esters thereof, and also acrylamide or derivatives thereof, vinyl ethers and vinylpyrrolidone;
   - polystyrenesulfonic acid salts, sodium salts, with a molecular mass of about 500 000 and of about 100 000. These compounds are described in patent FR 2 198 719;
   - polyacrylamidesulfonic acid salts such as those mentioned in patent US 4 128 631.
G) grafted anionic silicone polymers.
   The grafted silicone polymers used are preferably chosen from polymers containing a non-silicone organic backbone grafted with monomers containing a polysiloxane, polymers containing a polysiloxane backbone grafted with non-silicone organic monomers, and mixtures thereof.
H) anionic polyurethanes, which may comprise silicone grafts and silicones bearing hydrocarbon-based grafts.

Examples of fixing polyurethanes that may especially be mentioned include the dimethylolpropionic acid/isophorone diisocyanate/neopentyl glycol/polyester diols copolymer (also known under the name polyurethane-1, INCI name) sold under the brand name Luviset^{®} Pur by the company BASF, and the dimethylolpropionic acid/isophorone diisocyanate/neopentyl glycol/polyester diols/silicone diamine copolymer (also known under the name polyurethane-6, INCI name) sold under the brand name Luviset^{®} Si PUR A by the company BASF.

Another anionic polyurethane that may also be used is Avalure UR 450.

Polymers containing sulfoisophthalate groups, such as the polymers AQ55 and AQ48 sold by the company Eastman, may also be used.

According to the invention, the anionic polymers are preferably chosen from acrylic acid copolymers such as the acrylic acid/ethyl acrylate/N-tert-butylacrylamide terpolymer sold under the name Ultrahold Strong^{®} by the company BASF, and methacrylic acid/ethyl acrylate copolymers, especially in aqueous dispersion, such as Luviflex Soft and Luvimer MAE sold by the company BASF. Crotonic acid-based copolymers such as vinyl acetate/vinyl tert-butylbenzoate/crotonic acid terpolymers and crotonic acid/vinyl acetate/vinyl neododecanoate terpolymers sold under the name Resin 28-29-30 by the company National Starch, polymers derived from maleic, fumaric or itaconic acids or anhydrides with vinyl esters, vinyl ethers, vinyl halides or phenylvinyl derivatives, acrylic acid and esters thereof, such as the methyl vinyl ether/monoesterified maleic anhydride copolymer sold under the name Gantrez^{®} ES 425 by the company ISP, Luviset Si Pur, Mexomer PW, elastomeric or non-elastomeric anionic polyurethanes, and polymers containing sulfoisophthalate groups.

The cationic fixing polymers that may be used according to the present invention are preferably chosen from polymers comprising primary, secondary, tertiary and/or quaternary amine groups forming part of the polymer chain or directly attached thereto, and having a molecular weight of between 500 and approximately 5 000 000 and preferably between 1000 and 3 000 000.

Among these polymers, mention may be made more particularly of the following cationic polymers:
(1) homopolymers or copolymers derived from acrylic or methacrylic esters or amides and comprising at least one of the units of the following formulae: 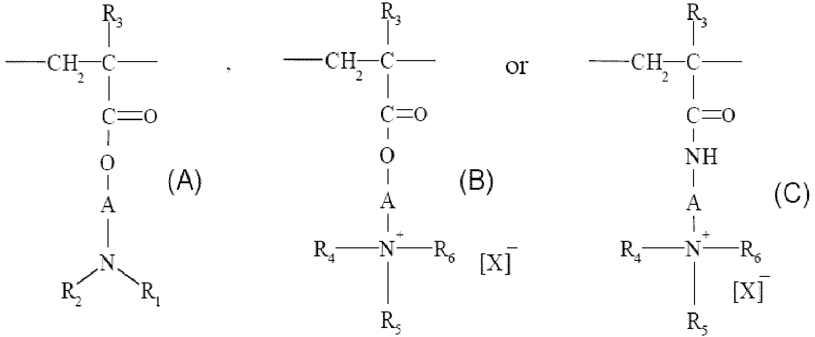 in which:
   R₃ denotes a hydrogen atom or a CH₃ group;
   A is a linear or branched alkyl group comprising from 1 to 6 carbon atoms or a hydroxyalkyl group comprising from 1 to 4 carbon atoms;
   R₄, R₅ and R₆, which are identical or different, represent an alkyl group containing from 1 to 18 carbon atoms or a benzyl group;
   R₁ and R₂, which may be identical or different, each represent a hydrogen atom or an alkyl group containing from 1 to 6 carbon atoms;
   X denotes a methosulfate anion or a halide such as chloride or bromide.
   The copolymers of family (1) also contain one or more units deriving from comonomers that may be chosen from the family of acrylamides, methacrylamides, diacetone acrylamides, acrylamides and methacrylamides which are substituted on the nitrogen with C₁-C₄ alkyl groups, groups derived from acrylic or methacrylic acids or esters thereof, vinyllactams such as vinylpyrrolidone or vinylcaprolactam, and vinyl esters.
   Thus, among these copolymers of family (1), mention may be made of:
   - copolymers of acrylamide and of dimethylaminoethyl methacrylate quaternized with dimethyl sulfate or with a dimethyl halide, such as the product sold under the name Hercofloc^{®} by the company Hercules,
   - copolymers of acrylamide and of methacryloyloxyethyltrimethylammonium chloride, described, for example, in patent application EP-A-080 976 and sold under the name Bina Quat P 100 by the company Ciba Geigy,
   - copolymers of acrylamide and of methacryloyloxyethyltrimethylammonium methosulfate, such as the product sold under the name Reten by the company Hercules,
   - quaternized or non-quaternized vinylpyrrolidone/ dialkylaminoalkyl acrylate or methacrylate copolymers, such as the products sold under the name Gafquat^{®} by the company ISP, such as, for example, Gafquat^{®} 734 or Gafquat^{®} 755, or alternatively the products known as Copolymer^{®} 845, 958 and 937. These polymers are described in detail in French patents 2 077 143 and 2 393 573,
   - fatty-chain polymers containing a vinylpyrrolidone unit, such as the products sold under the name Styleze W20 and Styleze W10 by the company ISP,
   - dimethylaminoethyl methacrylate/ vinylcaprolactam/ vinylpyrrolidone terpolymers, such as the product sold under the name Gaffix VC 713 by the company ISP, and
   - quaternized vinylpyrrolidone/ dimethylaminopropyl-methacrylamide copolymers, such as the products sold under the name Gafquat^{®} HS 100 by the company ISP;
(2) cationic guar gums, preferably containing quaternary ammonium, such as those described in US patents 3 589 578 and 4 031 307, such as guar gums containing trialkylammonium cationic groups. Such products are sold in particular under the trade names Jaguar C13S, Jaguar C 15 and Jaguar C 17 by Meyhall;
(3) quaternary copolymers of vinylpyrrolidone and of vinylimidazole;
(4) chitosans or salts thereof; the salts that can be used are, in particular, chitosan acetate, lactate, glutamate, gluconate or pyrrolidonecarboxylate.
   These compounds include the chitosan having a degree of deacetylation of 90.5% by weight which is sold under the name Kytan Brut Standard by the company Aber Technologies, and the chitosan pyrrolidone carboxylate which is sold under the name Kytamer^{®} PC by the company Amerchol.
(5) cationic cellulose derivatives such as copolymers of cellulose or of cellulose derivatives grafted with a water-soluble monomer comprising a quaternary ammonium, and described in particular in patent US 4 131 576, such as hydroxyalkylcelluloses, for instance hydroxymethyl-, hydroxyethyl- or hydroxypropylcelluloses grafted in particular with a methacryloyloxyethyltrimethylammonium, methacrylamidopropyltrimethylammonium or dimethyldiallyl-ammonium salt.

The commercial products corresponding to this definition are more particularly the products sold under the name Celquat L 200 and Celquat H 100 by the company National Starch.

The amphoteric fixing polymers that can be used in accordance with the invention can be chosen from polymers comprising units B and C distributed randomly in the polymer chain, in which B denotes a unit derived from a monomer comprising at least one basic nitrogen atom and C denotes a unit derived from an acid monomer comprising one or more carboxylic or sulfonic groups, or alternatively B and C can denote groups derived from carboxybetaine or sulfobetaine zwitterionic monomers; B and C can also denote a cationic polymer chain comprising primary, secondary, tertiary or quaternary amine groups, in which at least one of the amine groups bears a carboxylic or sulfonic group connected via a hydrocarbon group or alternatively B and C form part of a chain of a polymer containing an ethylenedicarboxylic unit in which one of the carboxylic groups has been made to react with a polyamine comprising one or more primary or secondary amine groups.

The amphoteric polymers corresponding to the definition given above that are more particularly preferred are chosen from the following polymers:
(1) polymers resulting from the copolymerization of a monomer derived from a vinyl compound bearing a carboxylic group such as, more particularly, acrylic acid, methacrylic acid, maleic acid, α-chloroacrylic acid, and a basic monomer derived from a substituted vinyl compound containing at least one basic atom, such as, more particularly, dialkylaminoalkyl methacrylates and acrylates, dialkylaminoalkylmethacrylamides and -acrylamides. Such compounds are described in patent US 3 836 537.
   The vinyl compound may also be a dialkyldiallylammonium salt such as diethyldiallylammonium chloride.
(2) polymers comprising units deriving from:
   a) at least one monomer chosen from acrylamides and methacrylamides substituted on the nitrogen with an alkyl group,
   b) at least one acidic comonomer containing one or more reactive carboxylic groups, and
   c) at least one basic comonomer such as acrylic and methacrylic acid esters containing primary, secondary, tertiary and quaternary amine substituents, and the product of quaternization of dimethylaminoethyl methacrylate with dimethyl or diethyl sulfate.
   The N-substituted acrylamides or methacrylamides that are more particularly preferred according to the invention are groups in which the alkyl groups contain from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.
   The acidic comonomers are chosen more particularly from acrylic acid, methacrylic acid, crotonic acid, itaconic acid, maleic acid and fumaric acid and alkyl monoesters, having 1 to 4 carbon atoms, or maleic or fumaric acids or anhydrides. The preferred basic comonomers are aminoethyl, butylaminoethyl, N,N'-dimethylaminoethyl and N-tert-butylaminoethyl methacrylates. The copolymers whose CTFA (4th edition, 1991) name is octylacrylamide/acrylates/butylaminoethyl methacrylate copolymer, such as the products sold under the name Amphomer^{®} or Lovocryl^{®} 47 by the company National Starch, are particularly used.
(3) crosslinked and alkylated polyamino amides partially or totally derived from polyamino amides of general formula: 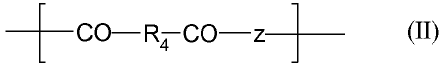 in which R₄ represents a divalent group derived from a saturated dicarboxylic acid, a mono- or dicarboxylic aliphatic acid bearing an ethylenic double bond, an ester of an alcohol containing 1 to 6 carbon atoms, of these acids, or a group derived from the addition of any one of the said acids to a bis(primary) amine or bis(secondary) derivative, and Z denotes a group derived from a bis(primary), mono- or bis(secondary) polyalkylene polyamine and preferably represents:
   a) in proportions of from 60 to 100 mol%, the group: 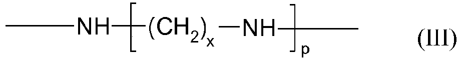 where x = 2 and p = 2 or 3, or alternatively x = 3 and p = 2
      this group being derived from diethylenetriamine, from triethylenetetramine or from dipropylenetriamine;
   b) in proportions of from 0 to 40 mol%, the group (III) above in which x = 2 and p = 1 and which is derived from ethylenediamine, or the group derived from piperazine: 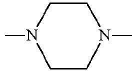
   c) in proportions of from 0 to 20 mol%, the -NH(CH₂)₆-NH-group derived from hexamethylenediamine, these polyamino amines being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof.
   The saturated carboxylic acids are preferably chosen from acids having 6 to 10 carbon atoms, such as adipic acid, 2,2,4-trimethyladipic acid and 2,4,4-trimethyladipic acid, terephthalic acid, acids containing an ethylenic double bond such as, for example, acrylic acid, methacrylic acid and itaconic acid. The alkane sultones used in the alkylation are preferably propane sultone or butane sultone, the salts of the alkylating agents are preferably the sodium or potassium salts.
(4) polymers comprising zwitterionic units of formula: 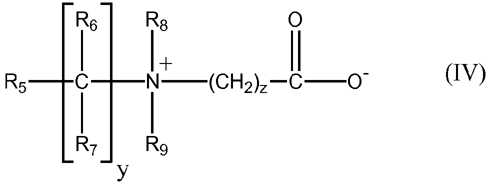 in which R₅ denotes a polymerizable unsaturated group such as an acrylate, methacrylate, acrylamide or methacrylamide group, y and z each represent an integer from 1 to 3, R₆ and R₇ represent a hydrogen atom, a methyl, ethyl or propyl group, R₈ and R₉ represent a hydrogen atom or an alkyl group such that the sum of the carbon atoms in R₁₀ and R₁₁ does not exceed 10.
   The polymers comprising such units can also comprise units derived from non-zwitterionic monomers such as dimethyl- or diethylaminoethyl acrylate or methacrylate or alkyl acrylates or methacrylates, acrylamides or methacrylamides or vinyl acetate.
(5) polymers derived from chitosan comprising monomer units corresponding to the following formulae: 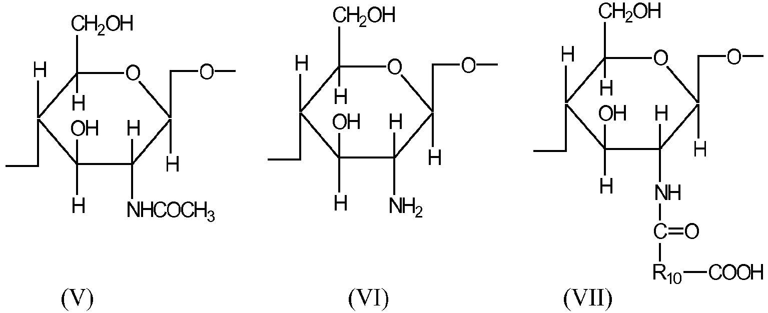 the unit (V) being present in proportions of between 0 and 30%, the unit (VI) in proportions of between 5% and 50% and the unit (VII) in proportions of between 30% and 90%, it being understood that, in this unit (VII), R₁₀ represents a group of formula: 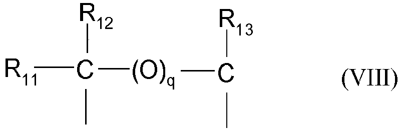
   in which, if q = 0, R₁₁, R₁₂ and R₁₃, which may be identical or different, each represent a hydrogen atom, a methyl, hydroxyl, acetoxy or amino residue, a monoalkylamine residue or a dialkylamine residue that are optionally interrupted by one or more nitrogen atoms and/or optionally substituted with one or more amine, hydroxyl, carboxyl, alkylthio or sulfonic groups, an alkylthio residue in which the alkyl group bears an amino residue, at least one of the groups R₁₁, R₁₂ and R₁₃ being, in this case, a hydrogen atom;
   or, if q = 1, R₁₁, R₁₂ and R₁₃ each represent a hydrogen atom, as well as the salts formed by these compounds with bases or acids.
(6) polymers derived from the N-carboxyalkylation of chitosan.
(7) polymers of units corresponding to the general formula (IX) described, for example, in French patent 1400 366: 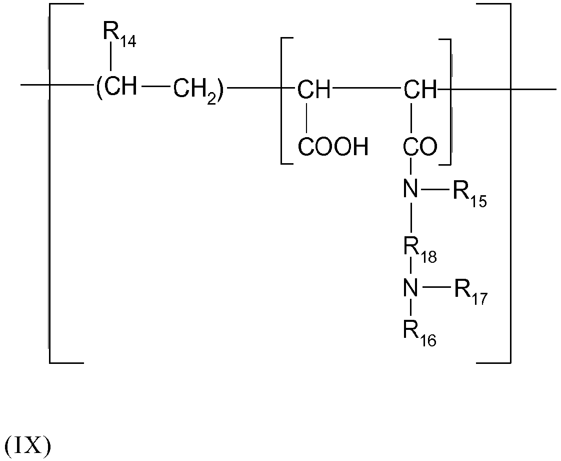 in which R₁₄ represents a hydrogen atom, a CH₃O, CH₃CH₂O or phenyl group, R₁₅ denotes a hydrogen atom or a C₁₋₄ alkyl group such as methyl or ethyl, R₁₆ denotes a hydrogen atom or a C₁₋₄ alkyl group such as methyl or ethyl, R₁₇ denotes a C₁₋₄ alkyl group such as methyl or ethyl or a group corresponding to the formula: -R₁₈-N(R₁₆)₂, R₁₈ representing a -CH₂-CH₂-, -CH₂-CH₂-CH₂- or -CH₂-CH(CH₃)- group, R₁₆ having the meanings mentioned above, and also the higher homologues of these groups, containing up to 6 carbon atoms.
(8) amphoteric polymers of the type -D-X-D-X- chosen from:
   a) polymers obtained by the action of chloroacetic acid or sodium chloroacetate on compounds comprising at least one unit of formula:

      -D-X-D-X-D- (X)

      where D denotes a 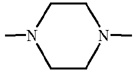 group and X denotes the symbol E or E', E or E', which may be identical or different, denote a divalent group that is an alkylene group with a straight or branched chain containing up to 7 carbon atoms in the main chain, which is unsubstituted or substituted with hydroxyl groups and which can comprise, in addition, oxygen, nitrogen and sulfur atoms and 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups.
   b) the polymers of formula:

      -D-X-D-X- (XI)

      where D denotes a 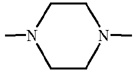 group and X denotes the symbol E or E' and at least once E'; E having the meaning given above and E' is a divalent group that is an alkylene group with a straight or branched chain having up to 7 carbon atoms in the main chain, which is unsubstituted or substituted with one or more hydroxyl groups and containing one or more nitrogen atoms, the nitrogen atom being substituted with an alkyl chain that is optionally interrupted by an oxygen atom and necessarily comprising one or more carboxyl functions or one or more hydroxyl functions and betainized by reaction with chloroacetic acid or sodium chloroacetate.
(9) (C₁-C₅)alkyl vinyl ether/maleic anhydride copolymers partially modified by semiamidation with an N,N-dialkylaminoalkylamine such as N,N-dimethylaminopropylamine or by semiesterification with an N,N-dialkanolamine. These copolymers can also comprise other vinyl comonomers such as vinylcaprolactam.

According to one preferred embodiment of the invention, the amphoteric fixing polymers that may be used in the aerosol device according to the invention may be chosen from branched block copolymers comprising:
(a) nonionic units derived from at least one monomer chosen from C₁-C₂₀ alkyl (meth)acrylates, N-mono(C₂-C₁₂ alkyl)-(meth)acrylamides and N,N-di(C₂-C₁₂ alkyl)(meth)acrylamides,
(b) anionic units derived from at least one monomer chosen from acrylic acid and methacrylic acid, and
(c) polyfunctional units derived from at least one monomer comprising at least two polymerizable unsaturated functional groups,
and preferably having a structure consisting of hydrophobic blocks onto which are fixed, via polyfunctional units (c), a number of more hydrophilic blocks.

Preferably, the amphoteric polymers have at least two glass transition temperatures (Tg), at least one of which is greater than 20°C and the other is less than 20°C.

The preferred amphoteric polymers are polymers comprising units derived from:
a) at least one monomer chosen from acrylamides and methacrylamides substituted on the nitrogen with an alkyl group,
b) at least one acidic comonomer containing one or more reactive carboxylic groups, and
c) at least one basic comonomer such as acrylic and methacrylic acid esters containing primary, secondary, tertiary and quaternary amine substituents, and the product of quaternization of dimethylaminoethyl methacrylate with dimethyl or diethyl sulfate.

Mention may be made in particular of the polymers sold under the name Amphomer by the company National Starch.

The nonionic fixing polymers that may be used according to the present invention are selected, for example, from:
- polyalkyloxazolines;
- vinyl acetate homopolymers;
- vinyl acetate copolymers, such as, for example, copolymers of vinyl acetate and acrylic ester, copolymers of vinyl acetate and ethylene, or copolymers of vinyl acetate and maleic ester, for example dibutyl maleate,
- homopolymers and copolymers of acrylic esters, for instance copolymers of alkyl acrylates and of alkyl methacrylates, such as the products sold by the company Röhm & Haas under the names Primal^{®} AC-261 K and Eudragit^{®} NE 30 D, by the company BASF under the name 8845, or by the company Hoechst under the name Appretan^{®} N9212,
- copolymers of acrylonitrile and a nonionic monomer chosen, for example, from butadiene and alkyl (meth)acrylates, such as the products sold under the name CJ 0601 B by the company Röhm & Haas,
- styrene homopolymers;
- styrene copolymers, for instance copolymers of styrene and of an alkyl (meth)acrylate, such as the products Mowilith^{®} LDM 6911, Mowilith^{®} DM 611 and Mowilith^{®} LDM 6070 sold by the company Hoechst, and the products Rhodopas^{®} SD 215 and Rhodopas^{®} DS 910 sold by the company Rhône-Poulenc, copolymers of styrene, of alkyl methacrylate and of alkyl acrylate, copolymers of styrene and of butadiene, or copolymers of styrene, of butadiene and of vinylpyridine,
- polyamides,
- vinyllactam homopolymers such as vinylpyrrolidone homopolymers and such as the polyvinylcaprolactam sold under the name Luviskol^{®} Plus by the company BASF,
- vinyllactam copolymers such as a poly(vinylpyrrolidone/vinyllactam) copolymer sold under the trade name Luvitec^{®} VPC 55K65W by the company BASF, poly(vinylpyrrolidone/vinyl acetate) copolymers, such as those sold under the name PVPVA^{®} S630L by the company ISP, Luviskol^{®} VA 73, VA 64, VA 55, VA 37 and VA 28 by the company BASF; and poly(vinylpyrrolidone/vinyl acetate/vinyl propionate) terpolymers, for instance the product sold under the name Luviskol^{®} VAP 343 by the company BASF, and
- poly(vinyl alcohols).

The alkyl groups of the nonionic polymers mentioned above preferably have from 1 to 6 carbon atoms.

Preferably, the fixing polymer(s) used in the aerosol device according to the invention are nonionic fixing polymers, in particular nonionic fixing polymers chosen from vinyllactam homopolymers, such as vinylpyrrolidone homopolymers, polyvinylcaprolactam, and vinyllactam copolymers, such as a poly(vinylpyrrolidone/vinyllactam) copolymer, poly(vinylpyrrolidone/vinyl acetate) copolymers and poly(vinylpyrrolidone/vinyl acetate/vinyl propionate) terpolymers.

More preferentially, the nonionic fixing polymer(s) used in the aerosol device according to the invention are chosen from vinyllactam homopolymers, such as vinylpyrrolidone homopolymers and polyvinylcaprolactam. In one preferred variant of the invention, the fixing polymer is polyvinylcaprolactam.

The fixing polymer(s) are present in an amount preferably ranging from 0.1% to 10% by weight, better still from 0.5% to 8% by weight and even more preferentially from 1% to 5% by weight relative to the total weight of the composition.

As C₂₋₄ monoalcohol(s) that may be used in the aerosol device of the invention, mention may be made especially of ethanol or isopropanol, and better still ethanol.

The C₂₋₄ monoalcohol(s) are preferably present in an amount ranging from 10% to 70% by weight, better still from 15% to 60% by weight and even more preferentially from 20% to 50% by weight relative to the total weight of the composition.

The composition according to the invention may contain one or more additional organic solvents such as polyols, for instance glycerol, propylene glycol or polyethylene glycols.

It may also contain water.

Preferably, it contains less than 5% by weight of water relative to the total weight of the composition. Even more preferentially, it does not contain any added water. The composition is then said to be anhydrous.

Examples of propellants that may be used in the aerosol device of the present invention are liquefied gases such as dimethyl ether, 1,1-difluoroethane, or C₃₋₅ alkanes, for instance propane, isopropane, n-butane, isobutane or pentane, or compressed gases such as air, nitrogen or carbon dioxide, and mixtures thereof.

Mention may be made preferentially of C₃₋₅ alkanes and in particular propane, n-butane and isobutane, and mixtures thereof.

The propellant(s) are preferably present in an amount ranging from 10% to 90% by weight, better still from 15% to 80% by weight and even more preferentially from 20% to 75% by weight relative to the total weight of the composition.

The cosmetic composition contained in the aerosol device according to the invention may also contain one or more additives chosen from silicone derivatives in soluble, dispersed or micro-dispersed form, clays such as bentone and hectorite, silica, plasticizers, protective screening agents, acids, bases, nacres, glitter flakes and fragrances, permanent or temporary dyes, and treating active agents.

These additives may be present in the composition according to the invention in an amount ranging from 0 to 20% by weight, relative to the total weight of the composition.

A person skilled in the art will take care to select these optional additives and the amounts thereof so that they do not harm the properties of the cosmetic compositions of the present invention.

The compositions in accordance with the invention are conditioned in an aerosol device that is common in cosmetics.

The aerosol device serving for conditioning the composition of the invention may consist of an outer aerosol can which contains both the propellant(s) and the other ingredients of the composition in a single compartment. Preferably, the said device is equipped with a valve with an inner restriction of 0.3 to 3 mm and better still from 0.5 to 1.5 mm, an inner nozzle of 0.3 to 3 mm and better still from 0.5 to 1.5 mm, and an additional gas intake of 0.3 to 3 mm and better still from 0.5 to 1.5 mm. The device also comprises a push button which is equipped with a direct outlet orifice with a diameter of between 0.25 and 1 mm and preferably between 0.4 and 0.7 mm.

According to another variant, the aerosol device may be in two compartments, formed from an outer aerosol can comprising an inner bag hermetically welded to a valve. The various ingredients of the composition are introduced into the inner bag and a compressed gas is introduced between the bag and the can at a sufficient pressure to make the product come out in the form of a spray through a nozzle orifice. Preferably, the said device is equipped with a valve with an inner restriction of 0.3 to 3 mm and better still from 0.5 to 1.5 mm, and an inner nozzle of 0.3 to 3 mm and better still from 0.5 to 1.5 mm. The device also comprises a push button which is equipped with a direct outlet orifice with a diameter of between 0.25 and 1 mm and preferably between 0.4 and 0.7 mm. Such a device is sold, for example, under the name EP Spray by the company EP-Spray System SA. The said compressed gas is preferably used at a pressure of between 1 and 12 bar and better still between 9 and 11 bar.

The sprayed compositions are in the form of a spray.

The present invention also relates to a cosmetic treatment process, especially a process for shaping the hair and/or for holding the hairstyle, comprising the use of a composition as described above.

In particular, the process for shaping and/or holding the hairstyle comprises a step of applying to wet or dry hair a composition as defined previously vaporized from an aerosol device according to the invention, to be rinsed out or left in after an optional leave-in time or after optional drying.

Preferably, the cosmetic composition according to the invention is not rinsed off.

The cosmetic composition according to the invention may thus be used on wet or dry hair. Preferably, the cosmetic composition is applied to clean hair.

According to one particular embodiment of the invention, the application of the composition may be followed by drying at room temperature or at a temperature above 40°C.

The drying may be performed immediately after the application or after a leave-in time that may range from 1 minute to 30 minutes.

The present invention also relates to the use of the cosmetic composition defined above sprayed from the aerosol device according to the invention, for shaping the hair and/or holding the hairstyle, and especially for giving the hairstyle volume.

The example that follows serves to illustrate the invention.

### EXAMPLE

In the example that follows, all the amounts are indicated as weight percentages of product as active materials relative to the total weight of the composition.

The composition below according to the invention was prepared from the compounds indicated in the table below.

| | |
|---|---|
| Calcium carbonate* | 5.0% |
| Polyvinylcaprolactam ** | 2.0% |
| Fragrance | 0.1% |
| Ethanol | 29.9% |
| Isobutane | 60.0% |
| * Sold under the trade name AH Mikhart 40 by the company Provencale S.A. | |
| ** Sold under the trade name Luviskol Plus by the company BASF | |

This composition was then introduced into an aerosol device. The said device is equipped with a valve with an inner restriction of 0.8 mm, an inner nozzle of 0.6 mm, an additional gas intake of 0.4 mm and a push button with a direct outlet orifice of 0.49 mm. The composition was sprayed onto a head of hair.

After drying, it is found that volume and body are given to the fibres, while at the same time maintaining a natural look of the head of hair, without a helmet effect.

## Claims

1. Aerosol device containing a cosmetic composition comprising:
(i) from 0.1% to 15% by weight relative to the total weight of the composition, of one or more water-insoluble calcium salts, the one or more water-insoluble calcium salts including calcium carbonate and /or calcium stearate,
(ii) one or more fixing polymers,
(iii) one or more C₂₋₄ monoalcohols, and
(iv) one or more propellants.

2. Aerosol device according to Claim 1, **characterized in that** the water-insoluble calcium salt is calcium carbonate.

3. Aerosol device according to Claim 1 or 2, **characterized in that** the water-insoluble calcium salt is in the form of a powder comprising particles with a mean diameter from 2 to 50 µm.

4. Aerosol device according to any one of the preceding claims, **characterized in that** the water-insoluble calcium salt(s) are present in an amount ranging from 0.5% to 10% by weight and better still from 1% to 5% by weight relative to the total weight of the composition.

5. Aerosol device according to any one of the preceding claims, **characterized in that** the fixing polymer(s) are chosen from nonionic fixing polymers.

6. Aerosol device according to any one of the preceding claims, **characterized in that** the fixing polymer(s) are chosen from vinyllactam homopolymers, such as vinylpyrrolidone homopolymers, polyvinylcaprolactam, and vinyllactam copolymers, such as a poly(vinylpyrrolidone/vinyllactam) copolymer, poly(vinylpyrrolidone/ vinyl acetate) copolymers and poly(vinylpyrrolidone/vinyl acetate/vinyl propionate) terpolymers, and is preferably polyvinylcaprolactam.

7. Aerosol device according to any one of the preceding claims, **characterized in that** the fixing polymer(s) are present in an amount ranging from 0.1% to 10% by weight, preferably from 0.5% to 8% by weight and better still from 1% to 5% by weight relative to the total weight of the composition.

8. Aerosol device according to any one of the preceding claims, **characterized in that** the C₂₋₄ monoalcohol is ethanol.

9. Aerosol device according to any one of the preceding claims, **characterized in that** the C₂₋₄ monoalcohol(s) are present in an amount ranging from 10% to 70% by weight, better still from 15% to 60% by weight and even better still from 20% to 50% by weight relative to the total weight of the composition.

10. Aerosol device according to any one of the preceding claims, **characterized in that** the propellant(s) are chosen from C₃₋₅ alkanes.

11. Aerosol device according to any one of the preceding claims, **characterized in that** the propellant(s) are present in an amount ranging from 10% to 90% by weight, better still from 15% to 80% by weight and even better still from 20% to 75% by weight relative to the total weight of the composition.

12. Aerosol device according to any one of the preceding claims, **characterized in that** the composition also contains one or more polyols.

13. Aerosol device according to any one of the preceding claims, **characterized in that** the composition contains less than 5% by weight of water, and is preferably anhydrous.

14. Aerosol device according to any one of the preceding claims, **characterized in that** the composition comprises one or more additives chosen from silicone derivatives in soluble, dispersed or micro-dispersed form, clays such as bentone or hectorite, silica, plasticizers, protective screening agents, acids, bases, nacres, glitter flakes and fragrances, permanent or temporary dyes, and treating active agents.

15. Process for shaping the hair and/or for holding the hairstyle, comprising a step of applying to wet or dry hair a cosmetic composition sprayed from an aerosol device according to any one of Claims 1 to 14, to be rinsed out or left in after an optional leave-in time or after optional drying.

16. Use of the cosmetic composition sprayed from the aerosol device according to any one of Claims 1 to 14, for shaping the hair and/or holding the hairstyle.

## Patentansprüche

1. Aerosolvorrichtung, die eine kosmetische Zusammensetzung enthält, die Folgendes umfasst:
(i) 0,1 bis 15 Gew.-%, bezogen auf das Gesamtgewicht der Zusammensetzung, eines oder mehrerer wasserunlöslicher Calciumsalze, wobei das wasserunlösliche Calciumsalz bzw. die wasserunlöslichen Calcium-Salze Calciumcarbonat und/oder Calciumstearat umfasst,
(ii) ein oder mehrere fixierende Polymere,
(iii) einen oder mehrere C₂₋₄-Monoalkohole und
(iv) ein oder mehrere Treibmittel.

2. Aerosolvorrichtung nach Anspruch 1, **dadurch gekennzeichnet, dass** es sich bei dem wasserunlöslichen Calciumsalz um Calciumcarbonat handelt.

3. Aerosolvorrichtung nach Anspruch 1 oder 2, **dadurch gekennzeichnet, dass** das wasserunlösliche Calciumsalz in Form eines Pulvers, das Teilchen mit einem mittleren Durchmesser von 2 bis 50 µm umfasst, vorliegt.

4. Aerosolvorrichtung nach einem der vorhergehenden Ansprüche, **dadurch gekennzeichnet, dass** das wasserunlösliche Calciumsalz bzw. die wasserunlöslichen Calcium-Salze in einer Menge im Bereich von 0,5 bis 10 Gew.-% und noch besser von 1 bis 5 Gew.-%, bezogen auf das Gesamtgewicht der Zusammensetzung, vorliegt bzw. vorliegen.

5. Aerosolvorrichtung nach einem der vorhergehenden Ansprüche, **dadurch gekennzeichnet, dass** das fixierende Polymer bzw. die fixierenden Polymere aus nichtionischen fixierenden Polymeren ausgewählt ist bzw. sind.

6. Aerosolvorrichtung nach einem der vorhergehenden Ansprüche, **dadurch gekennzeichnet, dass** das fixierende Polymer bzw. die fixierenden Polymere aus Vinyllactam-Homopolymeren, wie Vinylpyrrolidon-Homopolymeren, Polyvinylcaprolactam, und Vinyllactam-Copolymeren, wie einem Poly(vinylpyrrolidon/vinyllactam)-Copolymer, Poly(vinylpyrrolidon/vinylacetat)-Copolymeren und Poly(vinylpyrrolidon/vinylacetat/vinylpropionat)-Terpolymeren ausgewählt ist bzw. sind und es sich dabei vorzugsweise um Polyvinylcaprolactam handelt.

7. Aerosolvorrichtung nach einem der vorhergehenden Ansprüche, **dadurch gekennzeichnet, dass** das fixierende Polymer bzw. die fixierenden Polymere in einer Menge im Bereich von 0,1 bis 10 Gew.-%, vorzugsweise 0,5 bis 8 Gew.-% und noch besser 1 bis 5 Gew.-%, bezogen auf das Gesamtgewicht der Zusammensetzung, vorliegt bzw. vorliegen.

8. Aerosolvorrichtung nach einem der vorhergehenden Ansprüche, **dadurch gekennzeichnet, dass** es sich bei dem C₂₋₄-Monoalkohol um Ethanol handelt.

9. Aerosolvorrichtung nach einem der vorhergehenden Ansprüche, **dadurch gekennzeichnet, dass** der bzw. die C₂₋₄-Monoalkohole in einer Menge im Bereich von 10 bis 70 Gew.-%, noch besser von 15 bis 60 Gew.-% und noch besser von 20 bis 50 Gew.-%, bezogen auf das Gesamtgewicht der Zusammensetzung, vorliegt bzw. vorliegen.

10. Aerosolvorrichtung nach einem der vorhergehenden Ansprüche, **dadurch gekennzeichnet, dass** das bzw. die Treibmittel aus C₃₋₅-Alkanen ausgewählt ist bzw. sind.

11. Aerosolvorrichtung nach einem der vorhergehenden Ansprüche, **dadurch gekennzeichnet, dass** das bzw. die Treibmittel in einer Menge im Bereich von 10 bis 90 Gew.-%, noch besser von 15 bis 80 Gew.-% und noch besser von 20 bis 75 Gew.-%, bezogen auf das Gesamtgewicht der Zusammensetzung, vorliegt bzw. vorliegen.

12. Aerosolvorrichtung nach einem der vorhergehenden Ansprüche, **dadurch gekennzeichnet, dass** die Zusammensetzung außerdem ein oder mehrere Polyole enthält.

13. Aerosolvorrichtung nach einem der vorhergehenden Ansprüche, **dadurch gekennzeichnet, dass** die Zusammensetzung weniger als 5 Gew.-% Wasser enthält und Vorzugsweise wasserfrei ist.

14. Aerosolvorrichtung nach einem der vorhergehenden Ansprüche, **dadurch gekennzeichnet, dass** die Zusammensetzung ein oder mehrere Additive, die aus Silikonderivaten in löslicher, dispergierter oder mikrodispergierter Form, Tonen wie Benton oder Hectorit, Siliciumdioxid, Weichmachern, Schutzfiltern, Säuren, Basen, Perlglanzstoffen, Glitzerflocken und Duftstoffen, permanenten oder temporären Farbstoffen und Behandlungswirkstoffen ausgewählt sind, umfasst.

15. Verfahren zum Gestalten des Haars und/oder zum Halten der Frisur, bei dem man auf feuchtes oder trockenes Haar eine aus einer Aerosolvorrichtung nach einem der Ansprüche 1 bis 14 gesprühte kosmetische Zusammensetzung aufbringt, die gegebenenfalls nach einer fakultativen Einwirkungszeit oder nach fakultativer Trocknung auszuspülen ist.

16. Verwendung der aus der Aerosolvorrichtung nach einem der Ansprüche 1 bis 14 gesprühten kosmetischen Zusammensetzung zum Gestalten des Haars und/oder zum Halten der Frisur.

## Revendications

1. Dispositif aérosol qui contient une composition cosmétique comprenant :
(i) 0,1 à 15 % en poids par rapport au poids total de la composition, d'un ou plusieurs sels de calcium insolubles dans l'eau, le(s) sel(s) de calcium insoluble(s) dans l'eau incluant le carbonate de calcium et/ou le stéarate de calcium,
(ii) un ou plusieurs polymères fixants,
(iii) un ou plusieurs monoalcools en C₂₋₄, et
(iv) un ou plusieurs agents propulseurs.

2. Dispositif aérosol selon la revendication 1, **caractérisé en ce que** le sel de calcium insoluble dans l'eau est le carbonate de calcium.

3. Dispositif aérosol selon la revendication 1 ou 2, **caractérisé en ce que** le sel de calcium insoluble dans l'eau se présente sous la forme de poudre comprenant des particules qui ont un diamètre moyen de 2 à 50 µm.

4. Dispositif aérosol selon l'une quelconque des revendications précédentes, **caractérisé en ce que** le(s) sel(s) de calcium insoluble(s) dans l'eau est ou sont présent(s) en une quantité allant de 0,5 à 10 % en poids, mieux de 1 à 5% par rapport au poids total de la composition.

5. Dispositif aérosol selon l'une quelconque des revendications précédentes, **caractérisé en ce que** le(s) polymère(s) fixant(s) est ou sont choisi(s) parmi les polymères fixants non ioniques.

6. Dispositif aérosol selon l'une quelconque des revendications précédentes, **caractérisé en ce que** le(s) polymère(s) fixant(s) est ou sont choisi(s) parmi les homopolymères de vinyllactame, tels que les homopolymères de vinylpyrrolidone, le polyvinylcaprolactame, et les copolymères de vinyllactame, tels qu'un copolymère poly(vinylpyrrolidone/ vinyllactame), les copolymères poly(vinylpyrrolidone/ acétate de vinyle), et les terpolymères poly(vinylpyrrolidone/acétate de vinyle/propionate de vinyle), et de préférence est le polyvinylcaprolactame.

7. Dispositif aérosol selon l'une quelconque des revendications précédentes, **caractérisé en ce que** le(s) polymère(s) fixant(s) est ou sont présent(s) en une quantité allant de 0,1 à 10 % en poids, de préférence de 0,5 à 8 % en poids, mieux de 1 à 5% par rapport au poids total de la composition.

8. Dispositif aérosol selon l'une quelconque des revendications précédentes, **caractérisé en ce que** le monoalcool en C₂₋₄ est l'éthanol.

9. Dispositif aérosol selon l'une quelconque des revendications précédentes, **caractérisé en ce que** le ou les monoalcools en C₂₋₄ est ou sont présent(s) en une quantité allant de 10 à 70 %, mieux de 15 à 60%, encore mieux de 20 à 50 % en poids par rapport au poids total de la composition.

10. Dispositif aérosol selon l'une quelconque des revendications précédentes, **caractérisé en ce que** l'agent ou les agents propulseur(s) est ou sont choisi(s) parmi les alcanes en C₃₋₅.

11. Dispositif aérosol selon l'une quelconque des revendications précédentes, **caractérisé en ce que** l'agent ou les agents propulseur(s) est ou sont présent(s) en une quantité allant de 10 à 90 %, mieux de 15 à 80%, encore mieux de 20 à 75% en poids par rapport au poids total de la composition.

12. Dispositif aérosol selon l'une quelconque des revendications précédentes, **caractérisé en ce que** la composition contient en outre un ou plusieurs polyols.

13. Dispositif aérosol selon l'une quelconque des revendications précédentes, **caractérisé en ce que** la composition contient moins de 5% en poids d'eau, et est de préférence anhydre.

14. Dispositif aérosol selon l'une quelconque des revendications précédentes, **caractérisé en ce que** la composition comprend un ou plusieurs additifs choisis parmi les dérivés de silicones sous formes soluble, dispersée, micro-dispersée, des argiles telles que la bentone ou l'hectorite, de la silice, des plastifiants, des filtres protecteurs, des acides, des bases, des nacres, des paillettes et des parfums, des colorants permanents ou temporaires, et des actifs traitants.

15. Procédé de mise en forme des cheveux et/ou de maintien de la coiffure, comprenant une étape d'application sur les cheveux secs ou humides, d'une composition cosmétique pulvérisée à partir d'un dispositif aérosol selon l'une quelconque des revendications 1 à 14, à rincer ou non après un éventuel temps de pose ou après un éventuel séchage.

16. Utilisation de la composition cosmétique pulvérisée à partir du dispositif aérosol selon l'une quelconque des revendications 1 à 14, pour la mise en forme des cheveux et/ou le maintien de la coiffure.
